# EUROPEAN PATENT SPECIFICATION

(11) **EP 3 258 453 B1**
(45) Date of publication and mention of the grant of the patent: **06.01.2021**
(21) Application number: 17173742.2
(22) Date of filing: 31.05.2017
(51) Int. Cl.: A42B 3/04, G01D 21/00, G08B 21/02, G08B 21/12, A61B 5/00, A61B 5/024, A61B 5/053, A61B 5/11, G08B 21/04, G08B 25/01, G08B 25/10

(54) **SYSTEM AND METHOD FOR SENSING ENVIRONMENTAL CONDITIONS AND ALERTING A USER IN RESPONSE**
SYSTEM UND VERFAHREN ZUR ERFASSUNG VON UMWELTBEDINGUNGEN UND WARNEN EINES BENUTZERS ALS REAKTION
SYSTÈME ET PROCÉDÉS POUR DÉTECTER DES CONDITIONS ENVIRONNEMENTALES ET ALERTER UN UTILISATEUR EN RÉPONSE

(30) Priority: 10.06.2016 US 201615179397
(43) Date of publication of application: 20.12.2017
(73) Proprietor: The Boeing Company, Chicago, IL 60606-2016 (US)
(72) Inventor: GLATFELTER, John W., Chicago, IL 60606-2016 (US); LAUGHLIN, Brian D., Chicago, IL 60606-2016 (US)
(74) Representative: Boult Wade Tennant LLP

(56) References cited:
- US-A- 6 031 454
- US-A1- 2006 125 623
- US-B1- 9 013 297

## Description

### Technical Field

The present disclosure relates generally to workplace monitoring and, more particularly, to systems, methods, and apparatus for location-based monitoring of environmental conditions associated with a workplace.

### Background

Working environments often include a variety of activities occurring simultaneously, which can expose individuals to undesired conditions. Such working environments (e.g., a manufacturing environment) can present unique challenges for monitoring these conditions due to simultaneous existence of complex operations, workers and/or bystanders present in the environment, equipment within the environment, products within the environment, and processes within the environment, among other things, within the same working environment. Additional environmental factors, such as weather, terrain, ventilation, chemical and material presence, among other things, may also alter conditions of the working environment and introduce additional challenges.

These environmental factors of a working environment can create various effects on the environment, materials therein, and workers themselves. For efficiency and/or safety concerns, it is desired to effectively monitor such environmental conditions, as they relate to one or more workers within the working environment.

US-A-2006/0125623, in accordance with its abstract, states a system and method are disclosed for identifying monitoring and evaluating hazardous or potentially hazardous conditions. The system may be worn by safety personnel to detect equipment conditions such as low power supply, environmental conditions such as ambient temperature and/or physiological conditions such as heart rate of a wearer. The system may further include a control unit having electronics operable to communicate signals associated with equipment, environmental and physiological conditions.

US-B-6031454, in accordance with its abstract, states a person-specific monitor that provides sensor information regarding hazards to which the person is exposed and means to geolocate the person at the time of the exposure. The monitor also includes means to communicate with a remote base station. Information from the monitor can be downloaded at the base station for long term storage and analysis. The base station can also include means to recharge the monitor.

US-B-9013297, in accordance with its abstract, states a portable communications device transportable to remote locations by an individual includes a control unit. A biometric sensor is electrically connected to the control unit. The biometric sensor senses a condition of the individual and creates and transmits a signal to the control unit. An environmental sensor is electrically connected to the control unit. The environmental sensor senses an environmental condition local to the individual and creates and transmits a signal to the control unit. A communications unit is electrically connected to the control unit. It receives the signals from the control unit and transmits them away from the remote locations.

### Summary

There is described herein a system for monitoring one or more environmental conditions associated with a working environment, the system comprising: a first wearable device, the first wearable device configured to be worn by a first worker within the working environment and to move with the first worker within the working environment; one or more first environmental sampling sensors operatively associated with the first wearable device, each of the one or more first environmental sampling sensors configured to monitor one or more first environmental conditions associated with the working environment and determine first environmental sampling information based on the monitoring of the one or more first environmental conditions; a first positioning sensor operatively associated with the first wearable device and configured to determine first positioning information for the first worker, the first positioning information for the first worker including information regarding positioning of the first worker relative to the working environment; and a first controller, including a processor, configured to receive the first environmental sampling information from the one or more first environmental sampling sensors, receive the positioning information from the first positioning sensor, and determine, based on the first environmental sampling information and the first positioning information, if the one or more first environmental conditions necessitates an alert to be presented to the first worker; the system further comprising a second wearable device operatively associated with a second positioning sensor, configured to determine second positioning information, and a second controller, the second wearable device configured to be worn by a second worker within the working environment and move with the second worker within the working environment; a wireless network configured to provide communication amongst the first worker and the second worker via the first wearable device and second wearable device, respectively, and including one or more wireless transceivers associated with each of the first wearable device and the second wearable device, and wherein the second wearable device is further operatively associated with one or more second environmental sampling sensors, each of the one or more second environment sensors configured to monitor one or more second environmental conditions associated with the working environment relative to the second worker and determine second environmental sampling information based on the monitoring of the one or more environmental conditions, wherein the first controller is configured to receive the second positioning information and the second environmental sampling information from the second wearable device via the wireless network, wherein the first controller is further configured to determine positioning of the second worker based on the second positioning information, and wherein the first controller is further configured to determine, based on second environmental sampling information and the second positioning information, if the one or more second environmental conditions necessitates an aid alert be presented to the first worker, indicative of the second worker being in need of aid.

There is further described herein a method for monitoring environmental conditions associated with a working environment in relation to a worker within the working environment, the method comprising: monitoring one or more first environmental conditions using one or more first environmental sampling sensors, each of the one or more first environmental sampling sensors being operatively associated with a first wearable device configured to be worn by the first worker within the working environment; determining first environmental sampling information based on the monitoring of the one or more first environmental conditions using the one or more environmental sampling sensors; determining first positioning information for the first worker using a first positioning sensor operatively associated with the first wearable device, the first positioning information including information regarding positioning of the first worker relative to the working environment; receiving, by a first controller having a processor, the first environmental sampling information from the first environmental sampling sensors; receiving, by the first controller, the first positioning information from the first positioning sensor; determining, by the first controller, if the one or more first environmental conditions necessitates an alert be presented to the first worker, based on the first environmental sampling information and the first positioning information; and providing the alert to the first worker if the one or more first environmental conditions necessitates the alert be presented to the first worker; the method further comprising monitoring one or more second environmental conditions in relation to a second worker using one or more second environmental sampling sensors, each of the one or more second environmental sampling sensors being operatively associated with a second wearable device configured to be worn by the second worker within the working environment; determining second environmental sampling information based on the monitoring of the one or more second environmental conditions using the one or more second environmental sampling sensors; determining second positioning information for the second worker using a second positioning sensor operatively associated with the second wearable device, the second positioning information including information regarding positioning of the second worker relative to the working environment; receiving, by the first controller, the second positioning information and the second environmental sampling information from the second wearable device via a wireless network; determining, by the first controller, if the one or more second environmental conditions necessitates an aid alert be presented to the first worker, indicative of the second worker being in need of aid, based on the second environmental sampling information and the second positioning information; and presenting the aid alert to the first worker if the one or more second environmental conditions necessitates the aid alert be presented to the first worker.

In accordance with one example, a system for monitoring one or more environmental conditions associated with a working environment is disclosed. The system includes a wearable device configured to be worn by a worker within the working environment and to move with the worker within the working environment. The system further includes one or more environmental sampling sensors operatively associated with the wearable device, each of the one or more environmental sampling sensors configured to monitor the one or more environmental conditions associated with the working environment and determine environmental sampling information based on the monitoring of the one or more environmental conditions. The system further includes a positioning sensor operatively associated with the wearable device and configured to determine positioning information for the worker, the positioning information for the worker including information regarding positioning of the worker relative to the working environment. The system further includes a controller, which includes a processor. The controller is configured to receive the environmental sampling information from the one or more environmental sampling sensors, receive the positioning information from the positioning sensor, and determine, based on the environmental sampling information and the positioning information, if the one or more environmental conditions necessitates an alert to be presented to the worker.

In accordance with another example, a wearable device for monitoring environmental conditions associated with a working environment is disclosed. The wearable device includes a wearable article configured to be worn by a worker and to move with the worker. The wearable device further includes one or more modular environmental sampling sensors operatively associated with the wearable article, each of the one or more modular environment sensors configured to monitor the one or more environmental conditions associated with the working environment and determine environmental sampling information based on the monitoring of the one or more environmental conditions. The wearable device further includes a positioning sensor operatively affixed to the wearable article and configured to determine positioning information for the worker, the positioning information for the worker including information regarding positioning of the worker relative to the working environment. The wearable device further includes one or more output devices operatively associated with the wearable article and configured to receive an output signal and present a notification to the worker based on the output signal. The wearable device further includes a controller, which includes a processor, and is operatively associated with the modular environmental sampling sensors, the positioning sensor, and the one or more output devices. The controller is configured to receive the environmental sampling information from the one or more environmental sampling sensors, receive the positioning information from the positioning sensor, determine, based on the environmental sampling information and the positioning information, if the one or more environmental conditions necessitates an alert to be presented to the worker, and provide the output signal to the one or more output devices if the one or more environmental conditions necessitates the alert be presented to the worker.

In accordance with yet another example, a method for monitoring environmental conditions associated with a working environment in relation to a worker within the working environment is disclosed. The method includes monitoring the one or more environmental conditions using one or more environmental sampling sensors, each of the one or more environmental sampling sensors being operatively associated with a wearable device configured to be worn by the worker within the working environment. The method further includes determining environmental sampling information based on the monitoring of the one or more environmental conditions using the one or more environmental sampling sensors. The method further includes determining positioning information for the worker using a positioning sensor operatively associated with the wearable device, the positioning information including information regarding positioning of the worker relative to the working environment. The method further includes receiving, by a controller having a processor, the environmental sampling information from the environmental sampling sensors and receiving the positioning information from the positioning sensor. The method further includes determining, by the controller, if the one or more environmental conditions necessitates an alert be presented to the worker, based on the environmental sampling information and the positioning information and providing an alert to the worker if the one or more environmental conditions necessitates an alert be presented to the worker.

These and other aspects and features will become more readily apparent upon reading the following detailed description when taken in conjunction with the accompanying drawings. In addition, although various features are disclosed in relation to specific examples, it is understood that the various features may be combined with each other, or used alone, with any of the various examples.

### Brief Description of the Drawings

FIG. 1 is a schematic diagram of a system for monitoring one or more environmental conditions associated with a working environment, in accordance with the disclosure;
FIG. 2 is a schematic block diagram depicting elements of, or associated with, a wearable device used by the system of FIG. 1, in accordance with FIG. 1 and the disclosure;
FIG. 3 is a diagrammatic depiction of a wrist band for use as a wearable device, in accordance with the disclosure;
FIG. 4 is a diagrammatic depiction of a belt for use as a wearable device, in accordance with the disclosure;
FIG. 5, is a diagrammatic depiction of a modular sleeve for use as a wearable device, in accordance with the disclosure;
FIG. 6 is a diagrammatic depiction of headwear for use as a wearable device, in accordance with the disclosure;
FIG. 7 is a schematic diagram of a system for monitoring one or more environmental conditions associated with a working environment, in which multiple workers inhabit the working environment, in accordance with the disclosure;
FIG. 8 is an exemplary flowchart for a method for monitoring environmental conditions associated with a working environment in relation to a worker within the working environment, in accordance with the present disclosure; and
FIG. 9 is a schematic diagram for an exemplary computer that may execute instructions for providing the exemplary systems and methods of the present disclosure.

While the present disclosure is susceptible to various modifications and alternative constructions, certain illustrative examples thereof will be shown and described below in detail. The disclosure is not limited to the specific examples disclosed, but instead includes all modifications, alternative constructions, and equivalents thereof.

### Detailed Description

Reference will now be made in detail to specific examples or features, which are illustrated in the accompanying drawings. Generally, corresponding reference numbers will be used throughout the drawings to refer to the same or corresponding parts.

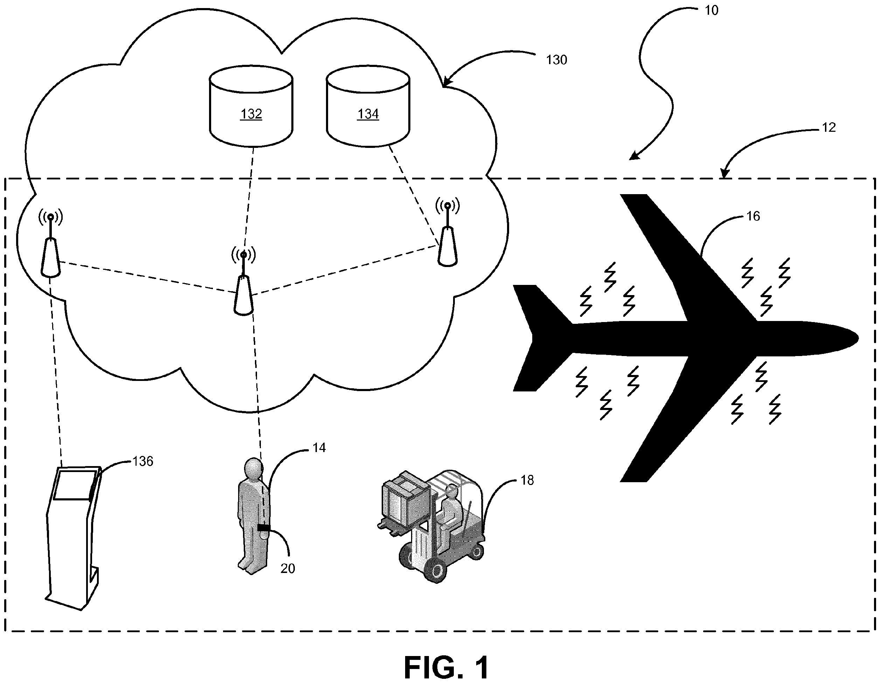

FIG. 1 illustrates an exemplary system 10 for monitoring environmental conditions of a working environment 12, with respect to a worker 14. The worker 14, as defined herein, is any human within the working environment 12 in any capacity (e.g., working, manufacturing, managing, observing, reporting, monitoring, resting, or any other active or passive activity). As defined herein, an "environmental condition with respect to the worker" may be any condition or occurrence within the working environment 12 that can affect the worker 14 in any way, be it a health related condition, a safety related condition, a proximity related condition, or any other condition. In one non-limiting example, environmental conditions include electrical feedback generated by operation of, or operation in service of, a machine 16. Another example environmental condition is proximity of the worker 14 to a mobile working machine 18. Further, in some examples, environmental conditions with respect to the user include environmental factors which can affect the health of the worker 14.

To participate in monitoring such environmental conditions within the working environment 12, the system 10 includes a wearable device 20. The wearable device 20 is configured to be worn by the human worker 14, while the worker 14 is within the working environment 12. As the wearable device 20 is to be worn by the worker 14 when performing any active or passive task within the working environment 12, positioning of the wearable device 20 is associated with positioning of the worker 14 within the working environment 12. The wearable device 20 can take the form of, or be affixed to, various wearable articles (jewelry, bracelets, headwear, accessories, eyewear, functional attire, etc.), as discussed in more detail below. Additionally, in some examples, the wearable device 20 is configured and/or configurable to communicate with other elements of the system 10 via any wired or wireless means, as discussed in more detail below.

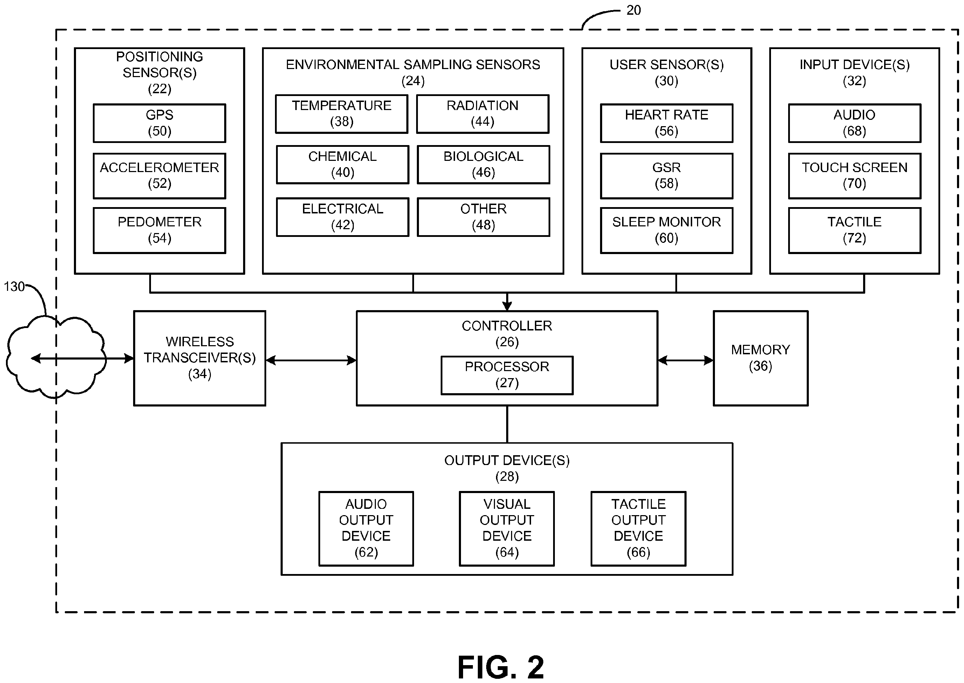

Turning to FIG. 2 and with continued reference to FIG. 1, a schematic diagram of the wearable device 20 is shown. While the elements of FIG. 2 are depicted as schematically encompassed by the wearable device 20, in some examples, some elements in the schematic depiction of the wearable device 20 are not physically located on or implemented as a part of the wearable device 20, but are, instead, operatively associated with the wearable device 20 and/or the worker 14 who is using the wearable device 20. As such, the schematic depiction of the wearable device 20 of FIG. 2 is intended to illustrate elements of or elements associated with the wearable device 20, which are used in conjunction to embody the systems and to perform the methods of the present disclosure.

The wearable device 20 includes, at least, positioning sensor(s) 22, environmental sampling sensor(s) 24, a controller 26, and output device(s) 28. In some examples, the wearable device 20 includes one or more of user sensor(s) 30, input device(s) 32, wireless transceiver(s) 34, and a memory 36. By utilizing the elements of the wearable device 20, the system 10 is capable of determining existence of one or more environmental conditions and positioning of the worker 14. In some examples, by using said information, the system 10 generates an output signal, if at least one of the one or more environmental conditions exists and it is deemed necessary to issue an alert to the worker 14, in response to the one or more environmental conditions.

The environmental sampling sensors 24 are operatively associated with the wearable device 20. The environmental sampling sensors 24 include one or more modular sensors configured to be added or removed from the system 10, based on system 10 need (e.g., "plug-and-play" type sensors). Utilizing modular sensors for environmental sampling sensors 24 may allow customizability for environmental condition monitoring performed by the system 10. Additionally or alternatively, the environmental sampling sensors 24 may include one or more sensors permanently affixed to the wearable device 20 and/or one or more of the environmental sampling sensors 24 may not be affixed to the wearable device 20 and may communicate with the wearable device 20, as he/she exists in the working environment 12. Further, in some examples, one or more of the environmental sampling sensors 24 may be sensors classified as "Internet of Things" (IoT) sensors.

Each of the environmental sampling sensors 24 are configured to monitor one or more environmental conditions associated with the working environment 12 and determine environmental sampling information based on such monitoring of the one or more environmental conditions. The environmental sampling sensors may operate independently, cooperatively, or both. For example, a temperature sensor 38 of the environmental sampling sensors 24 may monitor temperature-related (e.g., heat-based or cold-based) environmental conditions (e.g., heat or cold proximate to the worker 14), and said conditions may be indicative of a temperature-related environmental state within the working environment 12 that necessitates an alert be presented to the worker 14 (e.g., excessive heat or cold within the working environment 12 that may be harmful to the worker 14 and/or equipment within the working environment 12).

In some examples, the environmental sampling sensors 24 may also additionally or alternatively include chemical sensor(s) 40, which are configured to detect chemicals within the work environment and conditions related to chemicals as chemical-based environmental conditions that necessitate an alert be presented to the worker 14 (e.g., excessive parts per square inch of a chemical within the environment, excessive levels of chemicals in the atmosphere or ground, lack of oxygen, presence of carbon monoxide, excess of carbon dioxide, or any other chemical condition within the working environment 12).

In addition to or alternatively to the environmental sampling sensors described above, other example environmental sampling sensors 24 include, but are not limited to, electrical sensors 42, radiation sensors 44, biological sensors 46, and/or any other sensors 48. Electrical sensors 42 are configured to determine the presence of electrical conditions within the working environment 12 (e.g., electrical fields, electrical currents, at the like). Accordingly, electrical sensors 42 are configured to detect electric currents/fields within the work environment and conditions related to electricity and electrical-based environmental conditions that necessitate an alert be presented to the worker 14 (e.g. elevated electrical currents or fields in an environment, presence of exposed electrical wiring causing increases in electrical properties, and the like). In one example, the machine 16 of the working environment 12 may produce high electrical currents that are undesirable when in the presence of the worker 14. In such examples, the electrical sensor 42 may detect excessive electrical current in the working environment 12, or portions thereof, and alert the worker 14 to keep his/her distance from the machine 16.

In addition to or alternatively the environmental sampling sensors 24 may include the radiation sensor 44, the radiation sensor 44 is configured to determine the presence of radiation and/or radioactive materials and evaluate conditions associated with radiation and/or radioactive materials. Accordingly, the radiation sensor 44 determines one or both of immediate presence of radiation, with respect to the user, and cumulative presence of radiation, with respect to the worker 14. If either immediate or cumulative presence of radiation is at an unacceptable level, with respect to the worker 14, then such presence is a radiation-based environmental condition that necessitates an alert be presented to the worker 14.

To determine positioning information associated with the worker 14, the system 10 includes the positioning sensor(s) 22. The positioning sensors 22 sense the position of the worker 14 relative to the associated working environment 12. The positioning sensors 22 include one or more of individual sensors that cooperate to provide signals to the controller 26 to indicate the position of the worker 14 and/or determine characteristics of a motion of the worker 14, within the working environment 12. In some examples, the positioning sensor(s) 22 include one or more global positioning system (GPS) sensors 50 for detecting positioning of the worker 14 relative to the working environment 12. In some examples, the positioning sensors 22 include one or both of an accelerometer 52 and a pedometer 54. Of course, other elements aiding in detecting positioning of the worker 14 relative to the working environment 12 may be included.

To gather information associated with the worker 14 and, particularly, health of the worker 14, the system 10, in some examples, includes the user sensors 30. The user sensors 30 are any sensors configured for monitoring conditions directly associated with the worker 14, such as, but not limited to, health information associated with the worker 14. As such, the user sensors 30 are configured to generate health information associated with the worker 14.

In the non-limiting example presented herein, the user sensors 30 include a heart rate monitor 56, a galvanic skin response sensor (GSR) 58, and a sleep monitor 60, among other things. All such user sensors 30 are configured to capture data which is indicative of health conditions associated with the worker 14 (e.g., heart rate information from the heart rate monitor 56, electrodermal activity from the GSR sensor 58, sleep duration and/or sleep quality data from the sleep monitor 60, among other things). Of course, the health information gathered by the user sensors 30 is capable of including body temperature information for the worker 14, blood alcohol content (BAC) information of the worker 14, blood pressure of the worker 14, and any other health condition associated with the worker 14.

The controller 26, which includes a processor 27, receives as signals, at least, the environmental sampling information determined by the environmental sampling sensors 24 and the positioning information determined by the positioning sensors 22. Optionally, in some examples, the controller 26 receives the health information determined by the user sensors 30 or receives health information stored on a health information database. Based on the environmental sampling information, the positioning information, and, optionally, the health information, the controller 26 determines if the one or more environmental conditions necessitates an alert to be presented to the user via, for example, the output device(s) 28. For example, the environmental sampling information received by the controller 26 may indicate that the worker 14 is experiencing a high level of electrical current at his/her person and, based on the positioning information provided in conjunction with the environmental conditions, the controller 26 determines that the worker 14 is too close to the machine 16. Therefore, the controller 26 determines that the environmental conditions necessitate an alert for the worker 14. In some examples, the an output signal is provided by the controller 26 to the output devices 28, to provide the worker 14 with an alert regarding the environmental conditions (e.g., in the case of the electrical current, the controller, for example, indicates that the worker 14 is too close to the machine 16). In some examples, once the output device(s) 28 have issued the alert, the controller 26 receives an input signal, in response to the output signal, from the input device(s) 32, indicating that the worker 14 has received and/or acknowledged the alert.

The controller 26 is any electronic controller or computing system including a processor (e.g., the processor 27) which performs operations, executes control algorithms, stores data, retrieves data, gathers data, and/or performs any other computing or controlling task desired. The controller 26 may be a single controller or may include more than one controller disposed to control various functions and/or features of the system 10. Functionality of the controller 26 may be implemented in hardware and/or software and may rely on one or more data maps relating to functions of the system 10. To that end, the controller 26 may include internal memory 36 and/or the controller 26 may be otherwise connected to external memory, such as a database or server, via any wired or wireless networks. The memory 36 may include, but is not limited to including, one or more of read only memory (ROM), random access memory (RAM), a portable memory, and the like. Such memory media are examples of nontransitory memory media.

The controller 26 is capable of registering a location of the worker 14, using the positioning data and, optionally, time stamping said location data. The controller 26, in some examples, also can register the worker 14 in accordance with a current task. Further, the controller 26, in some examples, is configured to perform one or more of the following functions: authenticating the worker 14 for a task, clock the worker 14 on or off the working environment 12 or a task, check training records for the worker 14, and/or certifying the worker's qualifications for performing a task in the working environment 12. In such examples, such tasks are either performed at the controller 26, in communication with the memory 36, or the tasks are performed by the controller 26 in conjunction with one or more other controllers, databases, servers, and the like, which are in communication with the controller 26 via a network.

As discussed above, in some examples, if one or more environmental conditions are monitored and the controller 26 determines that said condition(s) necessitate an alert be presented to the worker 14, the alert is presented to the worker 14 via one or more output devices 28. The output devices 28 include one or more of an audio output device 62, a visual output device 64, a tactile output device 66, or a combination thereof. The audio output device 62 is any audio device capable of providing an audible signal to the worker 14 like, for example, a speaker. Such audible signals may be any audible noise of any amplitude, configured to alert the worker 14, in response to an output signal from the controller 26. The visual output device 64 is any light, screen, or visual device which may be configured to provide the worker 14 with any form of visual stimuli, in response to an output signal from the controller 26.

Further, the tactile output device 66 is any vibratory and/or haptic device configured to alert the worker 14 via one or more vibrations, in response to an operator alert signal. The tactile output device 66 is embedded in the wearable device 20, such that the tactile output device 66 will provide a vibratory alert to the worker 14 via the wearable device 20. Of course, additionally or alternatively, the tactile output device 66 may located anywhere within the working environment 12, wherein vibratory signals from the device will reach the worker 14. Additionally, output of the tactile output device 66 may be any vibration pattern, increasing or decreasing level of vibration, rhythmic vibration, or any other signifying vibration indicative of one or more environmental conditions.

In some examples of the system 10, it is desired that the worker 14 respond to the notification provided by the output device(s) 28. In such examples, the system 10 may further include one or more input device 32, configured to generate an input signal from the worker 14 in response to recognition, by the worker 14, of the notification signal. For example, the audio output device 62 may produce an audible signal telling the user "Stop," and, in response, an input device 32, such as a microphone 68, generates an input signal from spoken words by the worker 14, confirming he/she has stopped (e.g., the worker 14 responding to "Stop," from the audio output device 62, with "okay"). In some examples, the input devices 32 include one or more of the microphone 68, a touch screen 70, and tactile input sensor 72, among other possible input devices.

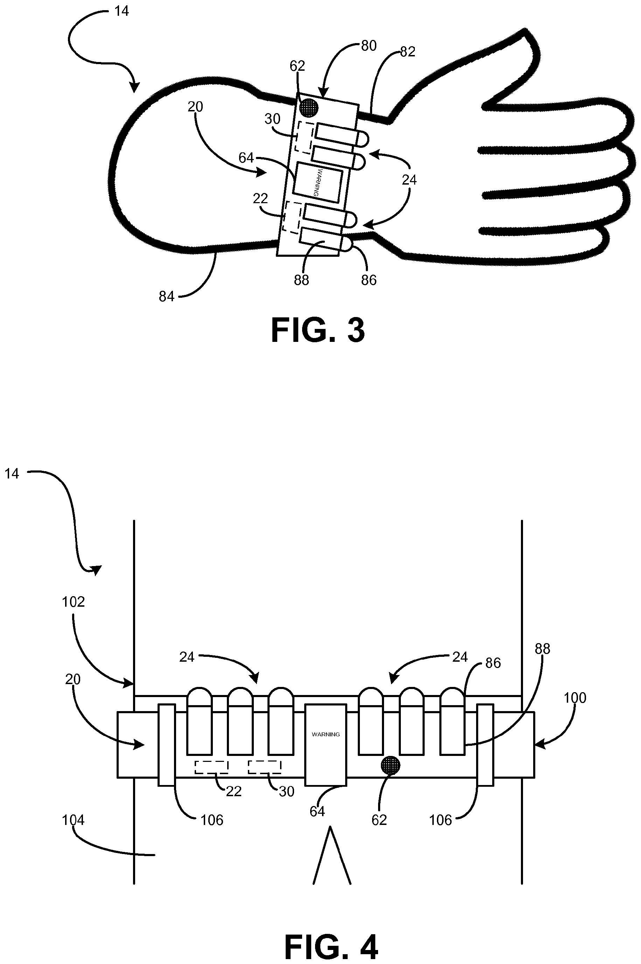

As discussed above, the wearable device 20 can take the form of, or be affixed to, various wearable articles. Examples of such wearable articles are illustrated in FIGS. 3-6 and described in more detail below. While the examples of FIGS. 3-6 illustrate examples directed towards four different wearable articles, it is certainly possible that the systems and methods of the present disclosure can be accomplished by utilizing any wearable article, on which the wearable device 20 is capable of being implemented.

Beginning with FIG. 3, a wrist band 80 is depicted, in which the wrist band 80 is implemented to embody, house, encase, or otherwise be functionally associated with the wearable device 20. The wearable device 20 is mounted to, attached to, embedded within, or otherwise affixed to the wrist band 80. As shown, the wrist band 80 is configured to be worn by the worker 14 on a wrist 82, or other portion, of an arm 84 of the worker 14.

Accordingly, the wrist band 80 is operatively associated with the position sensor(s) 22 of the wearable device 20, as to accurately track positioning of the worker 14 who is wearing the wrist band 80. Additionally, the wrist band 80 is operatively associated with one or more environmental sampling sensors 24. The environmental sampling sensors 24 may include modular sensors 86. In some examples, the modular sensors 86 are plugged into and taken out of modular sensor ports 88, enabling a "plug and play" aspect to the environmental sampling sensors 24. Additionally, output device(s) 28 of the wearable device 20 are operatively associated with the wrist band 80 and, for example, include a visual output device 64 and audio output device 62.

Turning now to FIG. 4, a belt 100 is depicted, in which the belt 100 is implemented to embody, house, encase, or be otherwise functionally associated with the wearable device 20. The wearable device 20 is mounted to, attached to, embedded within, or otherwise affixed to the belt. As shown, the belt 100 is configured to be worn by the worker 14 on a mid-section 102 (e.g., a waist, a natural waist, a chest, or any portion thereof). In some examples, the belt 100 is worn in conjunction with pants 104 that are worn by the worker 14 and the belt 100 is, optionally, supported by belt loops 106.

To track the positioning of the worker 14 who is wearing the belt 100, the belt 100 is operatively associated with the position sensor(s) 22 of the wearable device 20. Additionally, the belt 100 is operatively associated with one or more environmental sampling sensors 24. In some examples, the environmental sampling sensors 24 include modular sensors 86. In some examples, the modular sensors 86 are plugged into and taken out of modular sensor ports 88, enabling a "plug and play" aspect to the environmental sampling sensors 24. Additionally, output device(s) 28 of the wearable device 20 are operatively associated with the wrist band 80 and, for example, include a visual output device 64 and audio output device 62.

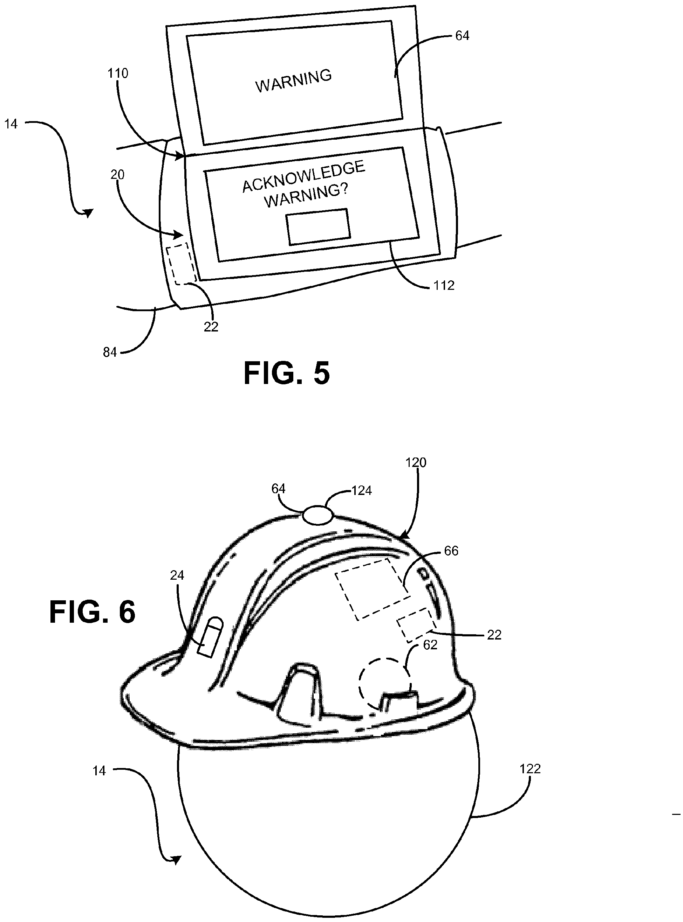

FIG. 5 illustrates yet another example of a wearable article for use with, or as, the wearable device 20, in which a modular sleeve 110 is depicted. The modular sleeve 110 is implemented to embody, house, encase, or otherwise be functionally associated with the wearable device 20. In some examples, the wearable device 20 is mounted to, attached to, embedded within, or otherwise affixed to the modular sleeve 110. As shown, the modular sleeve 110 is configured to be worn by the worker 14 on the arm 84 of the worker 14. The modular sleeve 110 is configured to expand or retract, in response to size of the arm 84 and/or changes in movement of the arm 84.

Accordingly, the modular sleeve 110 is operatively associated with the position sensor(s) 22 of the wearable device 20, as to accurately track positioning of the worker 14 who is wearing the modular sleeve 110. Additionally, the modular sleeve 110 is operatively associated with one or more environmental sampling sensors 24 (not shown). Further, output device(s) 28 of the wearable device 20 are operatively associated with the modular sleeve 110 and, for example, include a visual output device 64. The modular sleeve 110 may include input device(s) 32, such as a touch screen 112. The touch screen 112 is used to respond to and/or acknowledge alerts presented via the output device(s) 28, such as the visual output device 64.

In FIG. 6, headwear 120, such as a hardhat, bump-cap, or any other headwear, is depicted, in which the headwear 120 is implemented to embody, house, encase, or otherwise functionally associate with the wearable device 20. The wearable device 20 is mounted to, attached to, embedded within, or otherwise affixed to the headwear 120. As shown, the headwear 120 is configured to be worn by the worker 14 on a head 122 of the worker 14.

Accordingly, the headwear 120 is operatively associated with the position sensor(s) 22 of the wearable device 20, as to accurately track positioning of the worker 14 who is wearing the headwear 120. Additionally, the headwear 120 is operatively associated with one or more environmental sampling sensors 24. In some examples, the environmental sampling sensors 24 include modular sensors 86. In some examples, the modular sensors 86 are plugged into and taken out of modular sensor ports 88, enabling a "plug and play" aspect to the environmental sampling sensors 24. Further, the headwear 120, in some examples, is operatively associated with output devices 28. Some example output devices 28 for implementation with the headwear 120 include, but are not limited to including, an audio output device 62 positioned in proximity to an ear of the worker 14 when the worker 14 wears the headwear 120 on his/her head 122, a tactile output device 66 embedded within the headwear 120, and a visual output device 64, such as a light 124, configured to present visual stimuli to the worker 14 in response to an alert from the controller 26.

Returning now to FIG. 1, the wearable device 20 and/or the controller 26 are connected to other elements of the system 10 via a wireless network 130. The wireless network 130 is configured to connect the worker 14, via the wearable device 20, to any other workers 14 having their own wearable devices 20, to any databases, such as a user information database 132 and a user health information database 134, and to a worker kiosk 136, among other things. The worker kiosk is 136 is configured to check the worker 14 into the working environment 12 via the wearable device 20 and configured to store and transmit data via the wireless network 130.

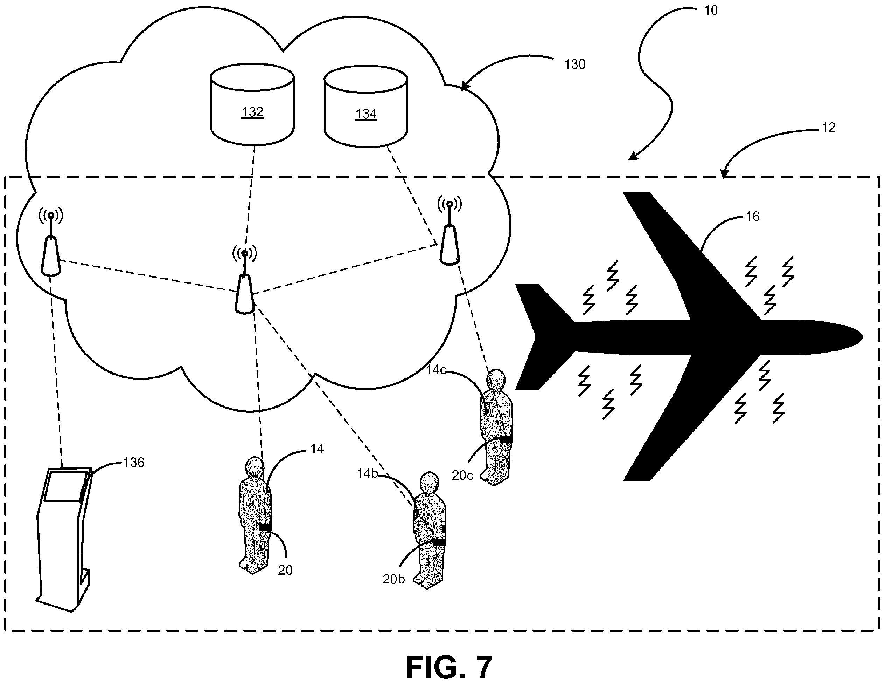

As discussed above, the working environment 12 often includes multiple workers 14 performing the same or different tasks. In such examples, like the example working environment 12 employing the system 10 of FIG. 7, multiple workers 14a, 14b, 14c are connected via their respective wearable devices 20a, 20b, 20c over the wireless network 130. Each of the wearable devices 20a, 20b, 20c are functionally embodied by the teachings of the wearable device 20, as detailed above. In such scenarios wherein multiple workers 14 are present at the working environment 12, each wearable device 20 can receive positioning information associated with other wearable devices 20. For example, the second worker 14b is wearing a second wearable device 20b and the second wearable device 20b provides positioning information to the controller 26 of the first wearable device 20. By receiving and utilizing such positioning information from the second wearable device 20b via the wireless network 130, the controller 26 of the first wearable device 20a determines positioning information associated with the second worker 14b. Additionally, the controller 26 can receive environmental sampling information from the second wearable device 20b, determined from environmental sampling sensors 24 of the second wearable device 20b. Utilizing positioning information and environmental sampling information from the second wearable device 20b, the controller 26 of the first wearable device 20a determines if environmental conditions affecting the second worker 14b necessitates that an aid alert be presented to the first worker 14a. The aid alert indicates that the second worker 14b is in need of aid (e.g., the second worker 14b is immobile, the second worker 14b is too close to a hazardous condition, etc.).

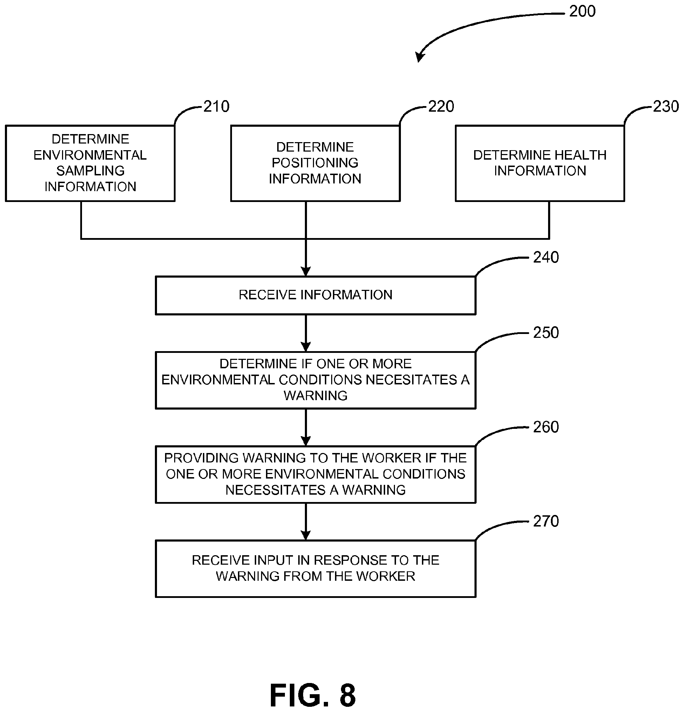

Turning now to FIG. 8, an example flowchart for a method 200 for monitoring environmental conditions associated with a working environment is shown. The method 200 will be described herein with reference to elements of the system 10 and their respective functions; however, the method 200 is certainly not limited to being implemented via the system 10 and its included elements.

The method 200 begins at blocks 210-230. At block 210, the environmental sampling sensors 24 monitor one or more environmental conditions and, based on said monitoring, determine environmental sampling information. At block 220, positioning information for the worker 14 is determined using the positioning sensor(s) 22, wherein the positioning information includes information regarding positioning of the worker 14 relative to the working environment 12. At the optional block 230, the method 200 includes determining health information for the worker 14 using one or more user sensors 30. Additionally or alternatively, in some examples, health information is provided from a database.

At block 240, the controller 26 receives the environmental sampling information, the positioning information and, optionally, the health information. Using the information received, the controller 26 determines if the one or more environmental conditions necessitates an alert be presented to the worker 14, as shown in block 250. If it is determined that the one or more environmental conditions necessitates an alert be presented to the worker 14, then an alert is provided to the worker 14 via, for example, one or more output devices 28, as shown in block 260. In some examples, the method 200 further includes receiving input in response to the alert from the worker 14 to, for example, acknowledge the alert was received, as shown in block 270.

It is to be understood that the flowchart of FIG. 8 is shown and described as an example only to assist in disclosing the features of the disclosed system and techniques, and that more or less steps than that shown may be included in the process corresponding to the various features described above for the disclosed system.

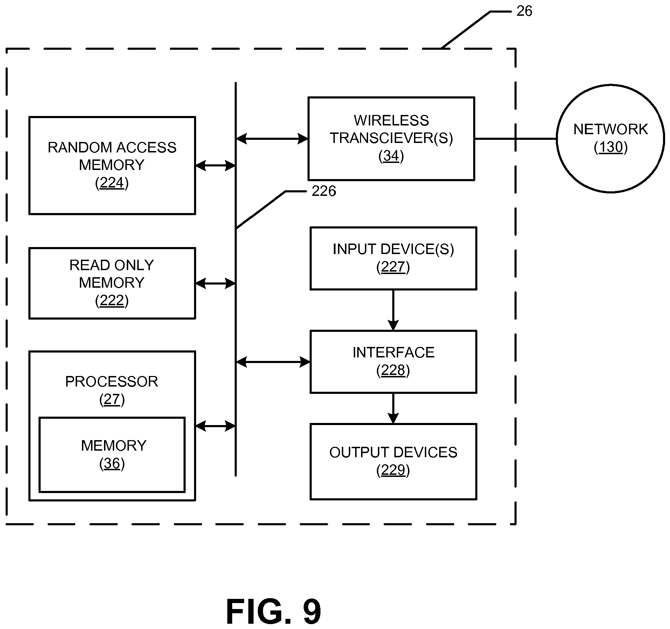

FIG. 9 schematically illustrates a combination of example elements which may be used to implement the controller 26 of the system 10, as shown in FIG. 2. The exemplary controller 26 is capable of executing instructions to realize the functions of the system 10 and/or execute instructions to perform the method 200, discussed above in reference to FIG. 8. The controller 26 may be, for example, a server, a personal computer, or any other type of computing device. The controller 26 of the instant example includes the processor 27. For example, the processor 27 may be implemented by one or more microprocessors or controllers from any desired family or manufacturer.

The processor 27 is associated with a memory 36 and is in communication with other memory including a read only memory 222 and a random access memory 224 via a bus 226. The random access memory 224 may be implemented by Synchronous Dynamic Random Access Memory (SDRAM), Dynamic Random Access Memory (DRAM), RAMBUS Dynamic Random Access Memory (RDRAM) and/or any other type of random access memory device. The read only memory 222 may be implemented by a hard drive, flash memory and/or any other desired type of memory device.

In some examples, the controller 26 includes an interface circuit 228. The interface circuit 228 may be implemented by any type of interface standard, such as, for example, an Ethernet interface, a universal serial bus (USB), and/or a PCI express interface. One or more input devices 227 are connected to the interface circuit 228. The input device(s) 227 permit a user to enter data and commands into the processor 27. The input device(s) 227 can be implemented by, for example, a keyboard, a mouse, a touchscreen, a track-pad, a trackball, and/or a voice recognition system. For example, the input device(s) 227 may include any wired or wireless device for connecting inputting data in to the controller 26 in response to an alert, as described above.

The output devices 229 are also connected to the interface circuit 228. The output devices 229 are implemented by any example output device (e.g., the output device(s) 28), as discussed above.

Further, in some examples, the controller 26 includes one or more wireless transceivers 34 for connecting to a network 130, such as the Internet, a WLAN, a LAN, a personal network, or any other network for connecting the controller 26 to one or more other controllers or network capable devices. As such, the controller 26 may be embodied by a plurality of controllers 26 for controlling various elements of the system 10.

As mentioned above the controller 26 may be used to execute machine readable instructions. For example, the controller 26 executes machine readable instructions to perform the method 200 shown in the block diagrams of FIG. 8. In such examples, the machine readable instructions comprise a program for execution by a processor, such as the processor 27, shown in the example controller 26. The program may be embodied in software stored on a tangible computer readable medium such as a CD-ROM, a floppy disk, a hard drive, a digital versatile disk (DVD), a Blu-ray disk, or a memory associated with controller 26, but the entire program and/or parts thereof could alternatively be executed by a device other than the processor 27 and/or embodied in firmware or dedicated hardware. Further, although the example programs are described with reference to the flowcharts illustrated in FIG. 8, many other methods of implementing examples of the present disclosure may alternatively be used. For example, the order of execution of the blocks may be changed, and/or some of the blocks described may be changed, eliminated, or combined.

## Claims

1. A system (10) for monitoring one or more environmental conditions associated with a working environment (12), the system comprising:
a first wearable device (20), the first wearable device (20) configured to be worn by a first worker (14) within the working environment (12) and to move with the first worker (14) within the working environment (12);
one or more first environmental sampling sensors (24) operatively associated with the first wearable device (20), each of the one or more first environmental sampling sensors (24) configured to monitor one or more first environmental conditions associated with the working environment (12) and determine first environmental sampling information based on the monitoring of the one or more first environmental conditions;
a first positioning sensor (22) operatively associated with the first wearable device (20) and configured to determine first positioning information for the first worker (14), the first positioning information for the first worker (14) including information regarding positioning of the first worker (14) relative to the working environment (12); and
a first controller (26), including a processor, configured to receive the first environmental sampling information from the one or more first environmental sampling sensors (24), receive the first positioning information from the first positioning sensor (22), and determine, based on the first environmental sampling information and the first positioning information, if the one or more first environmental conditions necessitates an alert to be presented to the first worker (14); the system further comprising
a second wearable device (20b) operatively associated with a second positioning sensor, configured to determine second positioning information, and a second controller, the second wearable device (20b) configured to be worn by a second worker (14b) within the working environment (12) and move with the second worker (14b) within the working environment (12);
a wireless network (130) configured to provide communication amongst the first worker (14) and the second worker (14b) via the first wearable device (20) and second wearable device (20b), respectively, and including one or more wireless transceivers (34) associated with each of the first wearable device (20) and the second wearable device (20b), and
wherein the second wearable device (20b) is further operatively associated with one or more second environmental sampling sensors (24), each of the one or more second environment sensors (24) configured to monitor one or more second environmental conditions associated with the working environment (12) relative to the second worker (14b) and determine second environmental sampling information based on the monitoring of the one or more environmental conditions,
wherein the first controller (26) is configured to receive the second positioning information and the second environmental sampling information from the second wearable device (20b) via the wireless network (130),
wherein the first controller (26) is further configured to determine positioning of the second worker (14b) based on the second positioning information, and
wherein the first controller (26) is further configured to determine, based on second environmental sampling information and the second positioning information, if the one or more second environmental conditions necessitates an aid alert be presented to the first worker (14), indicative of the second worker (14b) being in need of aid.

2. The system of claim 1, further comprising one or more output devices (28) operatively associated with the first wearable device (20) and the first controller (26) and configured to receive an output signal from the first controller (26) and present a notification to the first worker (14) based on the output signal, and
wherein the first controller (26) is further configured to generate the output signal if the one or more first environmental conditions necessitates the alert to be presented to the first worker (14), the system preferably further comprising one or more input devices 32 operatively associated with the first controller (26) and configured to generate an input signal based on input from the first worker (14),
wherein the first controller (26) is further configured to receive the input signal, the input signal being in response to the output signal.

3. The system of claim 2, wherein the one or more output devices (28) include at least one of a visual output device (64), an audio output device (62), a tactile output device (66), and any combinations thereof and the notification includes at least one of a visual notification, an audio notification, a tactile notification, and any combinations thereof.

4. The system of any one of claims 1 -3, wherein at least one of the one or more first environmental sampling sensors (24) is a modular sensor configured to be added and removed from the system.

5. The system of any one of claims 1-4, further comprising one or more user sensors (30) operatively associated with the first wearable device (20) and configured to generate health information associated with the first worker (14), and
wherein the first controller (26) is further configured to determine if the one or more first environmental conditions necessitates the alert to be presented to the first worker (14) based on the health information.

6. The system of any one of claims 1-5, further comprising a health information database (134) operatively associated with the first wearable device (20) and configured to store health information associated with the first worker (14), and
wherein the first controller (26) is further configured to determine if the one or more first environmental conditions necessitates the alert be presented to the first worker (14) based on the health information.

7. The system of any one of claims 1-6, wherein the one or more first environmental sampling sensors (24) include a chemical sensor configured to detect chemical-based environmental conditions, and
wherein the first controller (26) is configured to receive the chemical-based environmental conditions from the chemical sensor, and determine, based on the chemical-based environmental conditions and the first positioning information, if the chemical-based environmental conditions necessitates the alert be presented to the first worker (14).

8. The system of any one of claims 1-7, wherein the one or more first environmental sampling sensors (24) include an electrical sensor configured to detect electrical environmental conditions, and
wherein the first controller (26) is configured to receive the electrical environmental conditions from the electrical sensor, and determine, based on the electrical environmental conditions and the first positioning information, if the electrical environmental conditions necessitates the alert be presented to the first worker (14).

9. The system of any one of claims 1-8, wherein the one or more first environmental sampling sensors (24) include a temperature sensor configured to detect heat-based environmental conditions, and
wherein the first controller (26) is configured to receive the heat-based environmental conditions from the temperature sensor, and determine, based on the heat-based environmental conditions and the first positioning information, if the heat-based environmental conditions necessitates the alert be presented to the first worker (14).

10. The system of any one of claims 1-9, wherein the one or more first environmental sampling sensors (24) include a radiation sensor configured to detect radiation-based environmental conditions, and
wherein the first controller (26) is configured to receive the radiation-based environmental conditions from the radiation sensor, and determine, based on the radiation-based environmental conditions and the first positioning information, if the radiation-based environmental conditions necessitates the alert be presented to the first worker (14).

11. The system of claim 1, wherein the first wearable device (20) comprises:
a wearable article configured to be worn by the first worker (14) and to move with the first worker (14); and
one or more output devices (28) operatively associated with the wearable article and configured to receive an output signal and present a notification to the first worker (14) based on the output signal; wherein
the one or more first environmental sampling sensors (24) are modular and are operatively associated with the wearable article;
the first positioning sensor (22) is operatively affixed to the wearable article; and
the first controller (26) is operatively associated with the one or more modular environmental sampling sensors (24), the first positioning sensor (22), and the one or more output devices (28) and is configured to provide the output signal to the one or more output devices (28) if the one or more first environmental conditions necessitates the alert be presented to the first worker (14).

12. The system of claim 11, wherein the wearable article is
a wrist band (80) configured to be worn on an arm of the first worker (14),
a modular sleeve (110) configured to be worn on an arm of the first worker (14),
a belt (100) configured to be worn on a mid-section of the first worker (14), or
is headwear (120) configured to be worn on a head of the first worker (14).

13. A method for monitoring environmental conditions associated with a working environment (12) in relation to a worker (14) within the working environment (12), the method comprising:
monitoring one or more first environmental conditions using one or more first environmental sampling sensors (24), each of the one or more first environmental sampling sensors (24) being operatively associated with a first wearable device (20) configured to be worn by a first worker (14) within the working environment (12);
determining first environmental sampling information based on the monitoring of the one or more first environmental conditions using the one or more environmental sampling sensors (24);
determining first positioning information for the first worker (14) using a first positioning sensor (22) operatively associated with the first wearable device (20), the first positioning information including information regarding positioning of the first worker (14) relative to the working environment (12);
receiving, by a first controller (26) having a processor, the first environmental sampling information from the first environmental sampling sensors (24);
receiving, by the first controller (26), the first positioning information from the first positioning sensor (22);
determining, by the first controller (26), if the one or more first environmental conditions necessitates an alert be presented to the first worker (14), based on the first environmental sampling information and the first positioning information; and
providing the alert to the first worker (14) if the one or more first environmental conditions necessitates the alert be presented to the first worker (14); the method further comprising
monitoring one or more second environmental conditions in relation to a second worker (14b) using one or more second environmental sampling sensors, each of the one or more second environmental sampling sensors being operatively associated with a second wearable device (20b) configured to be worn by the second worker (14b) within the working environment (12);
determining second environmental sampling information based on the monitoring of the one or more second environmental conditions using the one or more second environmental sampling sensors (24);
determining second positioning information for the second worker (14b) using a second positioning sensor (22) operatively associated with the second wearable device (20b), the second positioning information including information regarding positioning of the second worker (14b) relative to the working environment (12);
receiving, by the first controller (26), the second positioning information and the second environmental sampling information from the second wearable device (20b) via a wireless network (130);
determining, by the first controller (26), if the one or more second environmental conditions necessitates an aid alert be presented to the first worker (14), indicative of the second worker (14b) being in need of aid, based on the second environmental sampling information and the second positioning information; and
presenting the aid alert to the first worker (14) if the one or more second environmental conditions necessitates the aid alert be presented to the first worker (14).

## Patentansprüche

1. System (10) zum Überwachen von ein oder mehreren mit einer Arbeitsumgebung (12) verknüpften Umgebungsbedingungen, wobei das System umfasst:
eine erste tragbare Vorrichtung (20), wobei die erste tragbare Vorrichtung (20) konfiguriert ist, um von einem ersten Arbeiter (14) in der Arbeitsumgebung (12) getragen zu werden und sich mit dem ersten Arbeiter (14) innerhalb der Arbeitsumgebung (12) zu bewegen;
einen oder mehrere erste Umgebungsmesssensoren (24), die mit der ersten tragbaren Vorrichtung (20) betreibbar verknüpft sind, wobei jeder der ein oder mehreren ersten Umgebungsmesssensoren (24) konfiguriert ist, um eine oder mehrere mit der Arbeitsumgebung (12) verknüpfte erste Umgebungsbedingungen zu überwachen und erste Umgebungsmessinformation auf der Grundlage der Überwachung der ein oder mehreren ersten Umgebungsbedingungen zu bestimmen;
einen ersten Positionssensor (22), der mit der ersten tragbaren Vorrichtung (20) betreibbar verbunden und konfiguriert ist, um erste Positionsinformation für den ersten Arbeiter (14) zu bestimmen, wobei die erste Positionsinformation für den ersten Arbeiter (14) Information bezüglich der Position des ersten Arbeiters (14) relativ zur Arbeitsumgebung (12) umfasst; und
eine erste Steuereinheit (26), einschließlich eines Prozessors, die konfiguriert ist, um die erste Umgebungsmessinformation von den ein oder mehreren ersten Umgebungsmesssensoren (24) zu empfangen, und auf der Grundlage der ersten Umgebungsmessinformation und der ersten Positionsinformation zu bestimmen, ob die ein oder mehreren ersten Umgebungsbedingungen es erfordern, dass dem ersten Arbeiter (14) ein Alarm angezeigt wird; wobei das System ferner umfasst
eine zweite tragbare Vorrichtung (20b), die betriebsfähig mit einem zweiten Positionssensor, der konfiguriert ist, um zweite Positionsinformationen zu bestimmen, und einer zweiten Steuereinheit verknüpft ist, wobei die zweite tragbare Vorrichtung (20b) konfiguriert ist, um von einem zweiten Arbeiter (14b) in der Arbeitsumgebung (12) getragen zu werden und sich mit dem zweiten Arbeiter (14b) in der Arbeitsumgebung (12) zu bewegen;
ein drahtloses Netzwerk (130), das konfiguriert ist, um eine Kommunikation zwischen dem ersten Arbeiter (14) und dem zweiten Arbeiter (14b) über die erste tragbare Vorrichtung (20) bzw. die zweite tragbare Vorrichtung (20b) bereitzustellen, und das einen oder mehrere drahtlose Sender-Empfänger (34) umfasst, die jeweils mit der ersten tragbaren Vorrichtung (20) und der zweiten tragbaren Vorrichtung (20b) verknüpft sind, und
wobei die zweite tragbare Vorrichtung (20b) ferner betreibbar mit einem oder mehreren zweiten Umgebungsmesssensoren (24) verknüpft ist, wobei jeder der ein oder mehreren zweiten Umgebungssensoren (24) konfiguriert ist, um eine oder mehrere zweite Umgebungsbedingungen zu überwachen, die mit der Arbeitsumgebung (12) in Bezug auf den zweiten Arbeiter (14b) verknüpft sind, und zweite Umgebungsmessinformation auf der Grundlage der Überwachung der einen oder mehreren Umgebungsbedingungen zu bestimmen,
wobei die erste Steuereinheit (26) konfiguriert ist, um die zweite Positionsinformation und die zweite Umgebungsmessinformation von der zweiten tragbaren Vorrichtung (20b) über das drahtlose Netzwerk (130) zu empfangen,
wobei die erste Steuereinheit (26) ferner konfiguriert ist, um die Position des zweiten Arbeiters (14b) auf der Grundlage der zweiten Positionsinformation zu bestimmen, und
wobei die erste Steuereinheit (26) ferner konfiguriert ist, um auf der Grundlage der zweiten Umgebungsmessinformation und der zweiten Positionsinformation zu bestimmen, ob die ein oder mehreren zweiten Umgebungsbedingungen es erfordern, dass dem ersten Arbeiter (14) ein Hilfsalarm angezeigt wird, der zeigt, dass der zweite Arbeiter (14b) Hilfe benötigt.

2. System nach Anspruch 1, das ferner eine oder mehrere Ausgabevorrichtungen (28) umfasst, die mit der ersten tragbaren Vorrichtung (20) und der ersten Steuereinheit (26) betreibbar verbunden und konfiguriert sind, um ein Ausgangssignal von der ersten Steuereinheit (26) zu empfangen und dem ersten Arbeiter (14) auf der Grundlage des Ausgangssignals eine Benachrichtigung anzuzeigen, und
wobei die erste Steuereinheit (26) ferner konfiguriert ist, um das Ausgangssignal zu erzeugen, wenn die ein oder mehreren ersten Umgebungsbedingungen es erforderlich machen, dass der Alarm dem ersten Arbeiter (14) angezeigt wird, wobei das System vorzugsweise ferner eine oder mehrere Eingabevorrichtungen (32) umfasst, die mit der ersten Steuereinheit (26) betreibbar verbunden sind und konfiguriert sind, um ein Eingangssignal basierend auf einer Eingabe von dem ersten Arbeiter (14) zu erzeugen,
wobei die erste Steuereinheit (26) ferner konfiguriert ist, um das Eingangssignal zu empfangen, wobei das Eingangssignal Reaktion auf das Ausgangssignal ist.

3. System nach Anspruch 2, bei dem die ein oder mehreren Ausgabevorrichtungen (28) mindestens eine visuelle Ausgabevorrichtung (64), eine Audio-Ausgabevorrichtung (62), eine taktile Ausgabevorrichtung (66) und/oder beliebige Kombinationen davon aufweisen und die Benachrichtigung eine visuelle Benachrichtigung, eine Audio-Benachrichtigung, eine taktile Benachrichtigung und/oder beliebige Kombinationen davon aufweist.

4. System nach einem der Ansprüche 1 bis 3, bei dem mindestens einer der ein oder mehreren ersten Umgebungsmesssensoren (24) ein modularer Sensor ist, der konfiguriert ist, um zu dem System hinzugefügt und aus ihm entfernt zu werden.

5. System nach einem der Ansprüche 1-4, ferner mit ein oder mehreren Benutzersensoren (30), die betreibbar mit der ersten tragbaren Vorrichtung (20) verbunden und konfiguriert sind, um mit dem ersten Arbeiter (14) verknüpfte Gesundheitsinformation zu erzeugen, und
bei dem die erste Steuereinheit (26) ferner konfiguriert ist, um auf der Grundlage der Gesundheitsinformation zu bestimmen, ob die ein oder mehreren ersten Umgebungsbedingungen es erforderlich machen, dass der Alarm dem ersten Arbeiter (14) angezeigt wird.

6. System nach einem der Ansprüche 1-5, das ferner eine Gesundheitsinformationsdatenbank (134) umfasst, die betreibbar mit der ersten tragbaren Vorrichtung (20) verbunden und konfiguriert ist, um mit dem ersten Arbeiter (14) verknüpfte Gesundheitsinformationen zu speichern, und
bei dem die erste Steuereinheit (26) ferner konfiguriert ist, um auf der Grundlage der Gesundheitsinformationen zu bestimmen, ob die ein oder mehreren ersten Umgebungsbedingungen es erfordern, dass der Alarm dem ersten Arbeiter (14) angezeigt wird.

7. System nach einem der Ansprüche 1-6, bei dem die ein oder mehreren ersten Umgebungsmesssensoren (24) einen chemischen Sensor umfassen, der konfiguriert ist, um auf Chemikalien basierende Umgebungsbedingungen zu erfassen, und
bei dem die erste Steuereinheit (26) konfiguriert ist, um die auf Chemikalien basierenden Umgebungsbedingungen von dem chemischen Sensor zu empfangen und auf der Grundlage der auf Chemikalien basierenden Umgebungsbedingungen und der ersten Positionsinformation zu bestimmen, ob die auf Chemikalien basierenden Umgebungsbedingungen es erforderlich machen, dass der Alarm dem ersten Arbeiter (14) angezeigt wird.

8. System nach einem der Ansprüche 1-7, bei dem die ein oder mehreren ersten Umgebungsmesssensoren (24) einen elektrischen Sensor umfassen, der konfiguriert ist, um elektrische Umgebungsbedingungen zu erfassen, und
wobei die erste Steuereinheit (26) konfiguriert ist, um die elektrischen Umgebungsbedingungen von dem elektrischen Sensor zu empfangen und auf der Grundlage der elektrischen Umgebungsbedingungen und der ersten Positionsinformation zu bestimmen, ob die elektrischen Umgebungsbedingungen es erfordern, dass der Alarm dem ersten Arbeiter (14) angezeigt wird.

9. System nach einem der Ansprüche 1-8, bei dem die ein oder mehreren ersten Umgebungsmesssensoren (24) einen Temperatursensor umfassen, der konfiguriert ist, um auf Wärme basierende Umgebungsbedingungen zu erfassen, und
wobei die erste Steuereinheit (26) konfiguriert ist, um die auf Wärme basierenden Umgebungsbedingungen von dem Temperatursensor zu empfangen und auf der Grundlage der auf Wärme basierenden Umgebungsbedingungen und der ersten Positionsinformation zu bestimmen, ob die auf Wärme basierenden Umgebungsbedingungen es erfordern, dass der Alarm dem ersten Arbeiter (14) angezeigt wird.

10. System nach einem der Ansprüche 1-9, bei dem die ein oder mehreren ersten Umgebungsmesssensoren (24) einen Strahlungssensor umfassen, der konfiguriert ist, um auf Strahlung basierende Umgebungsbedingungen zu erfassen, und
wobei die erste Steuereinheit (26) konfiguriert ist, um die auf Strahlung basierenden Umgebungsbedingungen von dem Strahlungssensor zu empfangen und auf der Grundlage der auf Strahlung basierenden Umgebungsbedingungen und der ersten Positionsinformation zu bestimmen, ob die auf Strahlung basierenden Umgebungsbedingungen es erforderlich machen, dass die Warnung dem ersten Arbeiter (14) angezeigt wird.

11. System nach Anspruch 1, bei dem die erste tragbare Vorrichtung (20) umfasst:
einen tragbaren Gegenstand, der konfiguriert ist, um von dem ersten Arbeiter (14) getragen zu werden und sich mit dem ersten Arbeiter (14) zu bewegen; und
eine oder mehrere Ausgabevorrichtungen (28), die mit dem tragbaren Gegenstand betreibbar verbunden und konfiguriert sind, um ein Ausgangssignal zu empfangen und dem ersten Arbeiter (14) auf der Grundlage des Ausgangssignals eine Benachrichtigung anzuzeigen; wobei
die ein oder mehreren ersten Umgebungsmesssensoren (24) modular sind und mit dem tragbaren Gegenstand betreibbar verbunden sind;
der erste Positionssensor (22) betreibbar an dem tragbaren Gegenstand befestigt ist; und
die erste Steuereinheit (26) mit dem einen oder den mehreren modularen Umgebungsmesssensoren (24), dem ersten Positionssensor (22) und den ein oder mehreren Ausgabevorrichtungen (28) betreibbar verbunden und konfiguriert ist, um das Ausgangssignal an die ein oder mehreren Ausgabevorrichtungen (28) zu liefern, wenn die ein oder mehreren ersten Umgebungsbedingungen es erfordern, dass der Alarm dem ersten Arbeiter (14) angezeigt wird.

12. System nach Anspruch 11, bei dem der tragbare Gegenstand ein Armband (80), das konfiguriert ist, um an einem Arm des ersten Arbeiters (14) getragen zu werden, eine modulare Manschette (110), die konfiguriert ist, um an einem Arm des ersten Arbeiters (14) getragen zu werden, ein Gürtel (100), der konfiguriert ist, um an einem Mittelabschnitt des ersten Arbeiters (14) getragen zu werden, oder eine Kopfbedeckung (120) ist, die konfiguriert ist, um an einem Kopf des ersten Arbeiters (14) getragen zu werden.

13. Verfahren zum Überwachen von Umgebungsbedingungen, die mit einer Arbeitsumgebung (12) verknüpft sind, in Bezug auf einen Arbeiter (14) in der Arbeitsumgebung (12), wobei das Verfahren umfasst:
Überwachen einer oder mehrerer erster Umgebungsbedingungen unter Verwendung eines oder mehrerer erster Umgebungsmesssensoren (24), wobei jeder der ein oder mehreren ersten Umgebungsmesssensoren (24) betriebsmäßig mit einer ersten tragbaren Vorrichtung (20) verknüpft ist, die konfiguriert ist, um von einem ersten Arbeiter (14) innerhalb der Arbeitsumgebung (12) getragen zu werden;
Bestimmen von ersten Umgebungsmessinformationen auf Grundlage der Überwachung der ein oder mehreren ersten Umgebungsbedingungen unter Verwendung der ein oder mehreren Umgebungsmesssensoren (24);
Bestimmen von erster Positionsinformation für den ersten Arbeiter (14) unter Verwendung eines ersten Positionssensors (22), der mit der ersten tragbaren Vorrichtung (20) betreibbar verknüpft ist, wobei die erste Positionsinformation Information bezüglich der Position des ersten Arbeiters (14) relativ zur Arbeitsumgebung (12) umfasst;
Empfangen der ersten Umgebungsmessinformation von den ersten Umgebungsmesssensoren (24) durch eine ersten Steuereinheit (26) mit einem Prozessor;
Empfangen der ersten Positionsinformation von dem ersten Positionssensor (22) durch die erste Steuereinheit (26);
Bestimmen, basierend auf der ersten Umgebungsmessinformationen und der ersten Positionsinformation, durch die erste Steuereinheit (26), ob die ein oder mehreren ersten Umgebungsbedingungen es erfordern, dass dem ersten Arbeiter (14) ein Alarm angezeigt wird; und
Anzeigen des Alarms für den ersten Arbeiter (14), wenn die ein oder mehreren ersten Umgebungsbedingungen es erfordern, dass der Alarm dem ersten Arbeiter (14) angezeigt wird; wobei das Verfahren ferner umfasst:
Überwachen von ein oder mehreren zweiten Umgebungsbedingungen in Bezug auf einen zweiten Arbeiter (14b) unter Verwendung von ein oder mehreren zweiten Umgebungsmesssensoren, wobei jeder der ein oder mehreren zweiten Umgebungsmesssensoren betreibbar mit einer zweiten tragbaren Vorrichtung (20b) verknüpft ist, die konfiguriert ist, um von dem zweiten Arbeiter (14b) innerhalb der Arbeitsumgebung (12) getragen zu werden;
Bestimmen von zweiten Umgebungsmessinformationen auf Grundlage der Überwachung der ein oder mehreren zweiten Umgebungsbedingungen unter Verwendung der ein oder mehreren zweiten Umgebungsmesssensoren (24);
Bestimmen der zweiten Positionsinformation für den zweiten Arbeiter (14b) unter Verwendung eines zweiten Positionssensors (22), der mit der zweiten tragbaren Vorrichtung (20b) betreibbar verknüpft ist, wobei die zweite Positionsinformation Informationen bezüglich der Position des zweiten Arbeiters (14b) relativ zur Arbeitsumgebung (12) umfasst;
Empfangen der zweiten Positionsinformation und der zweiten Umgebungsmessinformation von der zweiten tragbaren Vorrichtung (20b) über ein drahtloses Netzwerk (130) durch die erste Steuereinheit (26);
Bestimmen, basierend auf der zweiten Umgebungsmessinformation und der zweiten Positionsinformation, durch die erste Steuereinheit (26), ob die ein oder mehreren zweiten Umgebungsbedingungen erfordern, dass dem ersten Arbeiter (14) ein Hilfsalarm angezeigt wird, der zeigt, dass der zweite Arbeiter (14b) Hilfe benötigt; und
Anzeigen des Hilfsalarms für den ersten Arbeiter (14), wenn die ein oder mehreren zweiten Umgebungsbedingungen es erfordern, dass der Hilfsalarm dem ersten Arbeiter (14) angezeigt wird.

## Revendications

1. Système (10) destiné à surveiller une ou plusieurs conditions environnementales associées à un environnement de travail (12), le système comprenant :
un premier dispositif vestimentaire (20), le premier dispositif vestimentaire (20) étant configuré de manière à être porté par un premier travailleur (14) au sein de l'environnement de travail (12) et à se déplacer avec le premier travailleur (14) au sein de l'environnement de travail (12) ;
un ou plusieurs premiers capteurs d'échantillonnage environnemental (24) associés de manière fonctionnelle au premier dispositif vestimentaire (20), chaque capteur parmi ledit un ou lesdits plusieurs premiers capteurs d'échantillonnage environnemental (24) étant configuré de manière à surveiller une ou plusieurs premières conditions environnementales associées à l'environnement de travail (12), et à déterminer des premières informations d'échantillonnage environnemental sur la base de la surveillance de ladite une ou desdites plusieurs premières conditions environnementales ;
un premier capteur de positionnement (22) associé de manière fonctionnelle au premier dispositif vestimentaire (20), et configuré de manière à déterminer des premières informations de positionnement pour le premier travailleur (14), les premières informations de positionnement pour le premier travailleur (14) incluant des informations concernant le positionnement du premier travailleur (14) par rapport à l'environnement de travail (12) ; et
un premier contrôleur (26), incluant un processeur, configuré de manière à recevoir les premières informations d'échantillonnage environnemental en provenance dudit un ou desdits plusieurs premiers capteurs d'échantillonnage environnemental (24), à recevoir les premières informations de positionnement en provenance du premier capteur de positionnement (22), et à déterminer, sur la base des premières informations d'échantillonnage environnemental et des premières informations de positionnement, si ladite une ou lesdites plusieurs premières conditions environnementales nécessitent qu'une alerte soit présentée au premier travailleur (14) ; le système comprenant en outre :
un second dispositif vestimentaire (20b) associé de manière fonctionnelle à un second capteur de positionnement, configuré de manière à déterminer des secondes informations de positionnement, et un second contrôleur, le second dispositif vestimentaire (20b) étant configuré de manière à être porté par un second travailleur (14b) au sein de l'environnement de travail (12) et à se déplacer avec le second travailleur (14b) au sein de l'environnement de travail (12) ;
un réseau sans fil (130) configuré de manière à fournir une communication entre le premier travailleur (14) et le second travailleur (14b) par l'intermédiaire du premier dispositif vestimentaire (20) et du second dispositif vestimentaire (20b), respectivement, et incluant un ou plusieurs émetteurs-récepteurs sans fil (34) associés à chaque dispositif parmi le premier dispositif vestimentaire (20) et le second dispositif vestimentaire (20b) ; et
dans lequel le second dispositif vestimentaire (20b) est en outre associé de manière fonctionnelle à un ou plusieurs seconds capteurs d'échantillonnage environnemental (24), chaque capteur parmi ledit un ou lesdits plusieurs seconds capteurs d'échantillonnage environnemental (24) étant configuré de manière à surveiller une ou plusieurs secondes conditions environnementales associées à l'environnement de travail (12) par rapport au second travailleur (14b), et à déterminer des secondes informations d'échantillonnage environnemental sur la base de la surveillance de ladite une ou desdites plusieurs conditions environnementales ;
dans lequel le premier contrôleur (26) est configuré de manière à recevoir les secondes informations de positionnement et les secondes informations d'échantillonnage environnemental, en provenance du second dispositif vestimentaire (20b), par l'intermédiaire du réseau sans fil (130) ;
dans lequel le premier contrôleur (26) est en outre configuré de manière à déterminer le positionnement du second travailleur (14b) sur la base des secondes informations de positionnement ; et
dans lequel le premier contrôleur (26) est en outre configuré de manière à déterminer, sur la base de secondes informations d'échantillonnage environnemental et de secondes informations de positionnement, si ladite une ou lesdites plusieurs secondes conditions environnementales nécessitent qu'une alerte de secours soit présentée au premier travailleur (14), indiquant que le second travailleur (14b) a besoin d'assistance.

2. Système selon la revendication 1, comprenant en outre un ou plusieurs dispositifs de sortie (28) associés de manière fonctionnelle au premier dispositif vestimentaire (20) et au premier contrôleur (26), et configurés de manière à recevoir un signal de sortie en provenance du premier contrôleur (26) et à présenter une notification au premier travailleur (14) sur la base du signal de sortie ; et
dans lequel le premier contrôleur (26) est en outre configuré de manière à générer le signal de sortie, si ladite une ou lesdites plusieurs premières conditions environnementales nécessitent que l'alerte soit présentée au premier travailleur (14), le système comprenant de préférence en outre un ou plusieurs dispositifs d'entrée (32) associés de manière fonctionnelle au premier contrôleur (26), et configurés de manière à générer un signal d'entrée sur la base de l'entrée en provenance du premier travailleur (14) ;
dans lequel le premier contrôleur (26) est en outre configuré de manière à recevoir le signal d'entrée, le signal d'entrée étant en réponse au signal de sortie.

3. Système selon la revendication 2, dans lequel ledit un ou lesdits plusieurs dispositifs de sortie (28) incluent au moins un dispositif parmi un dispositif de sortie visuel (64), un dispositif de sortie audio (62), un dispositif de sortie tactile (66), et toute combinaison de ceux-ci, et la notification inclut au moins une notification parmi une notification visuelle, une notification audio, une notification tactile et toute combinaison de celles-ci.

4. Système selon l'une quelconque des revendications 1 à 3, dans lequel au moins l'un dudit un ou desdits plusieurs premiers capteurs d'échantillonnage environnemental (24) est un capteur modulaire configuré de manière à être ajouté au système et retiré du système.

5. Système selon l'une quelconque des revendications 1 à 4, comprenant en outre un ou plusieurs capteurs d'utilisateur (30) associés de manière fonctionnelle au premier dispositif vestimentaire (20) et configurés de manière à générer des informations de santé associées au premier travailleur (14) ; et
dans lequel le premier contrôleur (26) est en outre configuré de manière à déterminer si ladite une ou lesdites plusieurs premières conditions environnementales nécessitent que l'alerte soit présentée au premier travailleur (14) sur la base des informations de santé.

6. Système selon l'une quelconque des revendications 1 à 5, comprenant en outre une base de données d'informations de santé (134) associée de manière fonctionnelle au premier dispositif vestimentaire (20) et configurée de manière à stocker des informations de santé associées au premier travailleur (14) ; et
dans lequel le premier contrôleur (26) est en outre configuré de manière à déterminer si ladite une ou lesdites plusieurs premières conditions environnementales nécessitent que l'alerte soit présentée au premier travailleur (14) sur la base des informations de santé.

7. Système selon l'une quelconque des revendications 1 à 6, dans lequel ledit un ou lesdits plusieurs premiers capteurs d'échantillonnage environnemental (24) incluent un capteur chimique configuré de manière à détecter des conditions environnementales chimiques ; et
dans lequel le premier contrôleur (26) est configuré de manière à recevoir les conditions environnementales chimiques en provenance du capteur chimique, et à déterminer, sur la base des conditions environnementales chimiques et des premières informations de positionnement, si les conditions environnementales chimiques nécessitent que l'alerte soit présentée au premier travailleur (14).

8. Système selon l'une quelconque des revendications 1 à 7, dans lequel ledit un ou lesdits plusieurs premiers capteurs d'échantillonnage environnemental (24) incluent un capteur électrique configuré de manière à détecter des conditions environnementales électriques ; et
dans lequel le premier contrôleur (26) est configuré de manière à recevoir les conditions environnementales électriques en provenance du capteur électrique, et à déterminer, sur la base des conditions environnementales électriques et des premières informations de positionnement, si les conditions environnementales électriques nécessitent que l'alerte soit présentée au premier travailleur (14).

9. Système selon l'une quelconque des revendications 1 à 8, dans lequel ledit un ou lesdits plusieurs premiers capteurs d'échantillonnage environnemental (24) incluent un capteur de température configuré de manière à détecter des conditions environnementales thermiques ; et
dans lequel le premier contrôleur (26) est configuré de manière à recevoir les conditions environnementales thermiques en provenance du capteur de température, et à déterminer, sur la base des conditions environnementales thermiques et des premières informations de positionnement, si les conditions environnementales thermiques nécessitent que l'alerte soit présentée au premier travailleur (14).

10. Système selon l'une quelconque des revendications 1 à 9, dans lequel ledit un ou lesdits plusieurs premiers capteurs d'échantillonnage environnemental (24) incluent un capteur de rayonnement configuré de manière à détecter des conditions environnementales basées sur le rayonnement ; et
dans lequel le premier contrôleur (26) est configuré de manière à recevoir les conditions environnementales basées sur le rayonnement, en provenance du capteur de rayonnement, et à déterminer, sur la base des conditions environnementales basées sur le rayonnement et des premières informations de positionnement, si les conditions environnementales basées sur le rayonnement nécessitent que l'alerte soit présentée au premier travailleur (14).

11. Système selon la revendication 1, dans lequel le premier dispositif vestimentaire (20) comprend :
un article vestimentaire configuré de manière à être porté par le premier travailleur (14) et à se déplacer avec le premier travailleur (14) ; et
un ou plusieurs dispositifs de sortie (28) associés de manière fonctionnelle à l'article vestimentaire, et configurés de manière à recevoir un signal de sortie et à présenter une notification au premier travailleur (14), sur la base du signal de sortie ; dans lequel :
ledit un ou lesdits plusieurs premiers capteurs d'échantillonnage environnemental (24) sont modulaires et sont associés de manière fonctionnelle à l'article vestimentaire ;
le premier capteur de positionnement (22) est fixé de manière fonctionnelle à l'article vestimentaire ; et
le premier contrôleur (26) est associé de manière fonctionnelle audit un ou auxdits plusieurs capteurs d'échantillonnage environnemental modulaires (24), au premier capteur de positionnement (22), et audit un ou auxdits plusieurs dispositifs de sortie (28), et est configuré de manière à fournir le signal de sortie audit un ou auxdits plusieurs dispositifs de sortie (28), si ladite une ou lesdites plusieurs premières conditions environnementales nécessitent que l'alerte soit présentée au premier travailleur (14).

12. Système selon la revendication 11, dans lequel l'article vestimentaire est :
un bracelet (80) configuré de manière à être porté sur un bras du premier travailleur (14) ;
une manche élastique modulaire (110) configurée de manière à être portée sur un bras du premier travailleur (14) ;
une ceinture (100) configurée de manière à être portée sur une section médiane du premier travailleur (14) ; ou
un couvre-chef (120) configuré de manière à être porté sur une tête du premier travailleur (14).

13. Procédé de surveillance de conditions environnementales associées à un environnement de travail (12) en relation avec un travailleur (14) au sein de l'environnement de travail (12), le procédé comprenant les étapes ci-dessous consistant à :
surveiller une ou plusieurs premières conditions environnementales en utilisant un ou plusieurs premiers capteurs d'échantillonnage environnemental (24), chaque capteur parmi ledit un ou lesdits plusieurs premiers capteurs d'échantillonnage environnemental (24) étant associé de manière fonctionnelle à un premier dispositif vestimentaire (20) configuré de manière à être porté par un premier travailleur (14) au sein de l'environnement de travail (12) ;
déterminer des premières informations d'échantillonnage environnemental sur la base de la surveillance de ladite une ou desdites plusieurs premières conditions environnementales, en utilisant ledit un ou lesdits plusieurs capteurs d'échantillonnage environnemental (24) ;
déterminer des premières informations de positionnement pour le premier travailleur (14), en utilisant un premier capteur de positionnement (22) associé de manière fonctionnelle au premier dispositif vestimentaire (20), les premières informations de positionnement incluant des informations concernant le positionnement du premier travailleur (14) par rapport à l'environnement de travail (12) ;
recevoir, par le biais d'un premier contrôleur (26) présentant un processeur, les premières informations d'échantillonnage environnemental en provenance des premiers capteurs d'échantillonnage environnemental (24) ;
recevoir, par le biais du premier contrôleur (26), les premières informations de positionnement en provenance du premier capteur de positionnement (22) ;
déterminer, par le biais du premier contrôleur (26), si ladite une ou lesdites plusieurs premières conditions environnementales nécessitent qu'une alerte soit présentée au premier travailleur (14), sur la base des premières informations d'échantillonnage environnemental et des premières informations de positionnement ; et
fournir l'alerte au premier travailleur (14) si ladite une ou lesdits plusieurs premières conditions environnementales nécessitent que l'alerte soit présentée au premier travailleur (14) ; le procédé comprenant en outre les étapes ci-dessous consistant à :
surveiller une ou plusieurs secondes conditions environnementales en relation avec un second travailleur (14b), en utilisant un ou plusieurs seconds capteurs d'échantillonnage environnemental, chaque capteur parmi ledit un ou lesdits plusieurs seconds capteurs d'échantillonnage environnemental étant associé de manière fonctionnelle à un second dispositif vestimentaire (20b) configuré de manière à être porté par le second travailleur (14b) au sein de l'environnement de travail (12) ;
déterminer des secondes informations d'échantillonnage environnemental sur la base de la surveillance de ladite une ou desdites plusieurs secondes conditions environnementales, en utilisant ledit un ou lesdits plusieurs seconds capteurs d'échantillonnage environnemental (24) ;
déterminer des secondes informations de positionnement pour le second travailleur (14b), en utilisant un second capteur de positionnement (22) associé de manière fonctionnelle au second dispositif vestimentaire (20b), les secondes informations de positionnement incluant des informations concernant le positionnement du second travailleur (14b) par rapport à l'environnement de travail (12) ;
recevoir, par le biais du premier contrôleur (26), les secondes informations de positionnement et les secondes informations d'échantillonnage environnemental, en provenance du second dispositif vestimentaire (20b), par l'intermédiaire d'un réseau sans fil (130) ;
déterminer, par le biais du premier contrôleur (26), si ladite une ou lesdites plusieurs secondes conditions environnementales nécessitent qu'une alerte de secours soit présentée au premier travailleur (14), indiquant que le second travailleur (14b) a besoin d'assistance, sur la base des secondes informations d'échantillonnage environnemental et des secondes informations de positionnement ; et
présenter l'alerte de secours au premier travailleur (14), si ladite une ou lesdites plusieurs secondes conditions environnementales nécessitent que l'alerte de secours soit présentée au premier travailleur (14).
